# EUROPEAN PATENT SPECIFICATION

(11) **EP 1 958 617 B1**
(45) Date of publication and mention of the grant of the patent: **08.09.2010**
(21) Application number: 07380037.7
(22) Date of filing: 14.02.2007
(51) Int. Cl.: A61K 9/16

(54) **Pharmaceutical compositions containing quetiapine fumarate**
Pharmazeutische Zusammensetzungen mit Quetiapinfumarat
Compositions pharmaceutiques à base de fumarate de quétiapine

(43) Date of publication of application: 20.08.2008
(73) Proprietor: Laboratorios Lesvi, S.L., 08970 Sant Joan Despi (ES)
(72) Inventor: Ruiz Amenos, Anna, 08004 Barcelona (ES); Ubeda Pérez, Carmen, 08348 Cabrils, Barcelona (ES); Diez Martin, Ignacio, 08980 Sant Feliu de Llobregat, Barcelona (ES)
(74) Representative: ABG Patentes, S.L.

(56) References cited:
- WO-A-01/21179
- US-A1- 2005 158 383
- 20 April 2000 (2000-04-20), PFORMULATE , XP002440851 Retrieved from the Internet: URL:http://www.pformulate.com/glybehen.htm > [retrieved on 2007-07-04] * paragraph [0003] *

## Description

### FIELD OF THE INVENTION

The present invention relates to new pharmaceutical compositions for the oral administration of Quetiapine fumarate and to a process for its manufacture.

### BACKGROUND OF THE INVENTION

Quetiapine is a compound of formula (I): 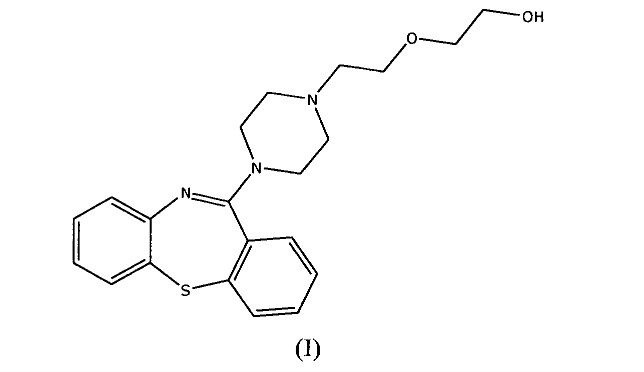 which has been employed as an antipsychotic or neuroleptic agent in the treatment of schizophrenia and bipolar mania, due to its antidopaminergic activity.

Quetiapine is currently marketed as a hemifumarate salt in the form of tablets of several doses of 25 mg, 100 mg, 200 mg and 300 mg for the administration two or three times per day. However, previously described formulations of quetiapine have certain drawbacks derived from the poor dissolution properties of this medicament and the uncontrolled release profile provided by said formulations. For example, document W02005/041935 describes Quetiapine formulations which do not provide a constant or substantially constant level of quetiapine, such that the patient can, at certain time intervals, receive therapeutic amounts of quetiapine exceeding the recommended doses, whereas at other times the amount may be below the therapeutically effective limits.

Patent applications WO97/45124 and W02005/041935 describe modified-release pharmaceutical compositions containing Quetiapine, i.e. they slowly release the active ingredient in long time intervals. For example patent WO2005/041935 describes solid dosage pharmaceutical compositions comprising a matrix formed by means of melted waxes, whereas application WO97/45124 describes the use of matrices with a gelling agent. However, in the later application, the use of water-soluble active ingredients, such as quetiapine or its pharmaceutically acceptable salts, combined with gelling agents such as hydroxypropylmethylcelluloses, can give rise to a phenomenon known as dumping in which the release of the active ingredient is delayed but once it starts the release occurs at very high rates.

Patent EP1218009 describes the preparation of granules containing Quetiapine and a freely or very water-soluble binder for their use in suspensions or solutions. Patent WO03/039516 relates to methods for improving the dissolution of poorly dispersible medicaments among them Quetiapine is included. The dissolution is improved by means of preparing granules in which a floating agent is added to the medicament. However, there is no indication about the release profile of these medicaments in the granulate formulations.

For all these reasons, there is still a need for developing pharmaceutical compositions which incorporate Quetiapine fumarate or methods for preparing said compositions with an improved physical stability which allows their marketing without the active ingredient release properties being affected.

### BRIEF DESCRIPTION OF THE INVENTION

The aim of the present invention is to provide pharmaceutical compositions containing quetiapine fumarate for oral administration having an improved dissolution profile and an improved physical stability without affecting the release profile of said active ingredient. In addition, it is an aim of the present invention to provide a process for preparing said pharmaceutical compositions, particularly tablets, by means of a process that can be applied at an industrial level with low energy costs and which does not subject the active ingredient to aggressive formulation conditions which can entail the loss of stability of the product.

The authors of the present invention have found that the use of granules containing quetiapine fumarate and coated with a lubricating agent allows preparing oral pharmaceutical compositions, particularly in the form of tablets, with an improved physical stability without affecting the dissolution properties of the oral compositions, making them suitable for therapeutic use.

Accordingly, a first aspect of the present invention is a granule for the preparation of pharmaceutical compositions comprising:
a) a core comprising quetiapine fumarate as active ingredient and a binder agent; and
b) a coating layer comprising a lubricant agent, wherein the lubricant agent is glyceryl behenate.

In a particular embodiment of the invention, the core may further comprise a diluent agent and/or a disintegrant agent.

A second aspect of the present invention is a process for preparing a granule as defined above comprising:
a) providing quetiapine fumarate and, optionally mixing it with a disintegrant agent and/or a diluent agent;
b) adding to the quetiapine fumarate or to the mixture obtained in step a) a binder agent;
c) adding a solvent to the mixture obtained in step b), or optionally, adding a solution or suspension containing a binder and a solvent to the quetiapine fumarate or to the mixture obtained in step a), thus suppressing step b);
d) wet granulating the mixture obtained in step c);
e) drying the granules obtained in step d);
f) sieving the dried granules obtained in step e); and
g) coating the granules with a lubricant agent, wherein the lubricant agent is glyceryl behenate.

Another aspect of the present invention relates to the use of the granule as defined above for the elaboration of pharmaceutical compositions.

Another aspect of the invention refers to a pharmaceutical composition comprising the granule as defined above, optionally in combination with one or more pharmaceutically acceptable excipients. In a particular embodiment, the pharmaceutical composition is in the form of a tablet.

Further, in another aspect the invention refers to an immediate release tablet which comprises the granule as defined above wherein the quantity of the lubricant agent is between 5 and 10% by weight with respect to the total weight of the granule.

Still in another aspect the invention relates to a sustained release tablet which comprises granules as defined above wherein the quantity of the lubricant agent is between 15 and 25% by weight with respect to the total weight of the granule.

Finally, another aspect of the invention is a process for the preparation of an immediate or sustained release tablet as defined above comprising:
a) preparing a granule as defined in claims 9 to 12;
b) optionally mixing the granule obtained in step a) with one or more pharmaceutically acceptable excipients;
c) tableting the granules obtained in step a) or in its case the mixture obtained in step b); and
d) coating the tablets obtained in step c).

### DETAILED DESCRIPTION OF THE INVENTION

In the present invention as "granule formulation" it is understood a set of granules, each of them comprising a core which comprises quetiapine fumarate as the active ingredient and a binder agent, said core being coated by a layer comprising a lubricating agent. In addition, the core of the granules may optionally comprise at least one diluent agent and/or a disintegrant agent.

Unless otherwise indicated, "quetiapine" is understood to be the compound quetiapine fumarate with a 2:1 stoichiometry, also known as quetiapine hemifumarate. Quetiapine may be incorporated in the granules in crystalline form either as a free compound or as a solvate. For example, in the case of using quetiapine fumarate, it can be incorporated in any of the several polymorphic forms described in patent applications WO99/06381, WO03/080065 and W02004/078735.

Quetiapine fumarate is preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the fumarate salt of the compound of formula (I), or of solvates or prodrugs.

The binder agent included in the core of the granules is selected from povidone, cornstarch, hydroxypropylcellulose and copovidone. Advantageously, the use of povidone K-25 as a binder is preferred. The amount of binder agent to be added to the core may vary between 1 and 15% by weight with respect to the total weight of the granule.

The diluent agent optionally included in the core is preferably selected from microcrystalline cellulose, lactose monohydrate and dibasic calcium phosphate. Advantageously, the use of microcrystalline cellulose as a diluent agent is particularly preferred. The amount of the diluent agent to be optionally added to the core may vary between 10 and 40% by weight with respect to the total weight of the granule.

The disintegrant agent optionally included in the core of the granule is preferably selected from sodium starch glycolate, crospovidone and sodium croscarmellose. Advantageously, sodium starch glycolate type A, known with the commercial name Primogel ®, as a disintegrant agent is particularly preferred. The amount of disintegrant agent to be optionally added to the core may vary between 3 and 20% by weight with respect to the total weight of the granule.

For coating the core of the granule, glyceryl behenate is used as the lubricating agent. The amount of lubricant agent to be used for preparing the coating layer may vary between 5 and 25% by weight with respect to the total weight of the granule.

In a particular embodiment of the invention, the granule is based on a set of granules, said granules comprise a core containing quetiapine hemifumarate combined with microcrystalline cellulose as a diluent agent, sodium starch glycolate (Primogel®) as a disintegrant agent and Povidone (PVP K25) as a binder, said core being coated with a glyceryl behenate layer as a lubricating agent.

The granules described above can be prepared by any method known in the state of the art. However, in a particular embodiment, said granule is prepared by wet granulation of quetiapine fumarate with a binder and optionally with a diluent agent and/or a disintegrant agent, followed by a coating process by means of a lubricant agent.

Accordingly, the process for preparing granules as described above comprises the steps of:
a) providing quetiapine fumarate and, optionally mixing it with a disintegrant agent and/or a diluent agent;
b) adding to the quetiapine fumarate or to the mixture obtained in step a) a binder agent;
c) adding a solvent to the mixture obtained in step b), or optionally, adding a solution or suspension containing a binder and a solvent to the quetiapine fumarate or to the mixture obtained in step a), thus suppressing step b);
d) wet granulating the mixture obtained in step c);
e) drying the granules obtained in step d);
f) sieving the dried granules obtained in step e); and
g) coating the granules with a lubricant agent, wherein the lubricant agent is glyceryl behenate.

The first step consists of providing quetiapine fumarate and optionally mixing the active ingredient quetiapine fumarate with a disintegrant and/or a diluent.

Quetiapine fumarate, can be prepared according to the method described in patent application WO2005/014590. The amount of quetiapine fumarate (expressed as quetiapine) in the granules is comprised between 20 and 80% of the total weight of the granule, preferably between 40 and 80%.

The amount of disintegrant to be optionally added is comprised between 3% and 20% by weight with respect to the total weight of the granule. Preferably, between 5% and 15% is added, more preferably between 7% and 12% of the total weight of the granule. The amount of diluent to be optionally added is comprised between 10% and 40% by weight with respect to the total weight of the granule, preferably between 15% and 25% of the total weight.

In the second step, the amount of binder to be added to the quetiapine fumarate or to the mixture obtained in step a) is comprised between 1 % and 15% by weight with respect to the total weight of the granule.

In the third step, a solvent is added in an amount comprised between 25% and 65% by weight with respect to the weight of mixture to be granulated, which will serve to carry out the wet mixture. Alternatively, the binder agent can be previously dissolved or suspended in said solvent and then added to the quetiapine fumarate or to the mixture obtained in step a) thus suppressing step b). As solvents for preparing the wet mixture, water, hydroalcoholic mixtures and alcohols can be used, being preferred the use of water as a solvent for the granulation of the mixture. This solvent is later eliminated from the composition by means of a drying step.

Then, in the fourth step, the wet granulation is performed. The granules can be produced by a known granulation method such as rolling granulation, fluidized-bed granulation, stirring granulation and the like. Additionally, suitable equipment for this type of processing can be used, for example, the granulation can be carried out in a low shear mixer or a high shear mixer. However, a low shear granulation will be preferably used since pharmaceutical compositions with a faster dissolution profile are obtained.

Once the wet granules are obtained, they are subjected to a drying process to eliminate the solvent. This step can be carried out for example in a fluid bed dryer. The granules are subjected to a temperature comprised between 40°C and 90°C, preferable between 60°C and 80°C, for the time period necessary to obtain granules with a moisture content less than 5%, preferably less than 3%.

Subsequently, the dried granules are calibrated by sieving or milling.

Finally, the sieved or milled granules are coated with glyceryl behenate. The coating process can be carried out by mixing the granules with the lubricating agent by any process known by a skilled person.

Surprisingly, the inventors have discovered that the amount of lubricating agent used for coating the granules allows controlling the rate of release of the active ingredient. Thus, the use of a lubricating agent as described above for coating the core of the granules in a proportion between 5% and 10% by weight with respect to the total weight of the granule allows preparing immediate release compositions. On the contrary, the use of the coating lubricating in a proportion between 15% and 25% by weight with respect to the total weight of the granule allows preparing sustained release compositions.

By "immediate release" it is understood a release form in which greater than or equal to about 50% or more, preferably about 75% of quetiapine is released within two hours of administration, preferably within one hour of administration.

By "sustained release" it is understood a release form in which quetiapine is released at such a rate that blood (e.g. plasma) levels are maintained within a therapeutic range but below toxic levels for at least 8 hours, preferably at least about 12 hours after administration.

Accordingly, the granule of the invention can be used for the elaboration of pharmaceutical compositions with different release profiles. Examples of these pharmaceutical compositions include any solid (tablets, pills, capsules, etc.) or liquid (solutions, suspensions or emulsions) composition for oral administration. In an embodiment of the invention, the pharmaceutical composition is an immediate release composition. In another embodiment, the pharmaceutical composition is a sustained release composition.

Suitable dose forms for oral administration may further contain conventional excipients known in the art such as binding agents, for example syrup, acacia, cellulose derivatives (i.e. hydroxypropylcellulose, carboxymethylcellulose, etc.) gelatin, sorbitol, tragacanth, or polyvinylpyrrolidone; fillers, for example lactose, sugar, maize starch, calcium phosphate, sorbitol or mannitol; tabletting lubricants, for example magnesium stearate; disintegrants, for example starch, crospovidone, sodium starch glycolate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The mentioned formulations will be prepared using standard methods such as those described or referred to in the Spanish and US Pharmacopoeias and similar reference texts.

In a preferred embodiment of the invention, the pharmaceutical composition is in the form of a tablet. The excipients used for preparing tablets may comprise between 0.25% and 5% by weight with respect to the total weight of the tablet of one or more lubricating agents, between 5% and 20% by weight of one or more disintegrants, between 20% and 50% by weight of one or more diluents and between 0.1% and 0.5% by weight of a glidant.

As disintegrant, low-substituted hydroxypropylcellulose, hydroxyethylcellulose, crospovidone, croscarmellose, starch, sodium carboxymethyl starch, casein derivatives or mixture thereof can be used.

As lubricating agent, magnesium stearate, calcium stearate, glyceryl palmitostearate, talcum, stearic acid, glyceryl behenate, sodium lauryl sulfate, sodium stearyl fumarate or mixtures thereof can be used. A stearate will preferably be used, still more preferably, magnesium stearate.

As diluent, a saccharide (monosaccharide or oligosaccharide, polysaccharides) and/or their oxidized and/or reduced forms; lactose in its anhydrous, monohydrate, agglomerated or spray forms; mannitol; cellulose powder, microcrystalline cellulose, silicified microcrystalline cellulose or chemically modified cellulose derivatives, such as hydroxypropylcellulose, hydroxypropylmethylcellulose; starch, sucrose, pharmaceutically acceptable inorganic compounds such as dibasic calcium phosphate, calcium or magnesium carbonates, magnesium oxide, or mixtures thereof can be used.

Additionally, previously prepared coprocessed diluents can be used such as Cellactose®, coprocessed lactose and cellulose powder, or Microcellac®, coprocessed lactose and microcrystalline cellulose, among others.

Preferably, cellulose will be used, more preferably, microcrystalline cellulose.

As glidant, anhydrous or hydrated colloidal silica, magnesium trisilicate or talc can be used.

The authors of the present invention have been able to prove that when quetiapine fumarate is mixed with the necessary excipients and are compressed directly with no other type of processing (e.g., granulation, etc..), the use of the direct compression method gives rise to very adherent tablets. It has also been proved that when quetiapine fumarate granules which are not coated with a lubricating agent are prepared, the obtained tablets are still adherent.

However, upon coating the granules with a lubricating agent, adhesions are minimized or disappear, providing tablets with a regular surface.

An additional aspect of the invention refers to an immediate release tablet which comprises granules as described previously wherein the quantity of lubricant agent is between 5% and 10% by weight with respect to the total weight of the granule. Further another aspect of the invention relates to a sustained release tablet which comprises granules as described previously wherein the quantity of lubricant agent is between 15% and 25% by weight with respect to the total weight of the granule.

Finally, another aspect of the present invention consists of providing a process for preparing an immediate or sustained release tablet as defined above which comprises:
1. preparing granules according to the process previously described;
2. optionally mixing the granules obtained in step 1) with one or more pharmaceutically acceptable excipients;
3. tableting the mixture obtained in step b); and
4. coating the tablets obtained in step c).

Step 1) comprises all the steps a) to g) previously described in the preparation of the granules of the invention. In the case of preparing an immediate release tablet, the amount of lubricating agent to be used for coating the core of the granules may vary between 5% and 10% by weight with respect to the total weight of the granule. However, in the case of preparing a sustained release tablet, the amount of lubricating agent to be used for coating the core of the granules may vary between 15% and 25% by weight with respect to the total weight of the granule

The excipients optionally used in step 2) for preparing tablets may comprise between 0.25% and 5% by weight with respect to the total weight of the tablet of one or more lubricating agents, between 5% and 20% by weight of one or more disintegrants, between 20% and 50% by weight of one or more diluents and between 0.1 % y un 0.5% by weight of a glidant.

The tableting process of step 3) can be carried out by any method known in the state of the art for preparing tablets, including for example direct compression, double compression, granulation etc. Finally, the tablet is coated with a conventional or enteric coating material or a polymeric coating material. For example, a mixture of hypromellose, titanium dioxide and macrogol in purified water can be used.

The pharmaceutical compositions of this invention may be used with other drugs to provide a combination therapy. The other drugs may form part of the same composition, or be provided as a separate composition for administration at the same time or at different time.

In the following, the present invention is further illustrated by examples. They should in no case be interpreted as a limitation of the scope of the invention as defined in the claims.

### Examples

### Example 1. Preparation of quetiapine fumarate tablets

Quantitative composition:

| | % |
|---|---|
| Quetiapine hemifumarate | 37 |
| Lactose monohydrate | 18 |
| Microcrystalline Cellulose | 18 |
| (Avicel PH102) | |
| Povidone (K-25) | 3 |
| Na starch glycolate type A (Primojel) | 15 |
| Glyceryl behenate | 5 |
| Anhydrous colloidal silica (Aerosil) | 0.3 |
| Magnesium stearate | 1 |
| Purified water* | 26* |
| Coating dispersion | 3** |

| | |
|---|---|
| * solvent which disappears during the manufacturing process. ** dry residue | |

Detailed description of the manufacturing process:
Quetiapine hemifumarate is mixed with povidone and 50% of the total sodium starch glycolate. The mixture is granulated in a low shear mixer with purified water, dried and sieved. The obtained granules are coated with glyceryl behenate by mixing. The coated granules are mixed with the remaining 50% of sodium starch glycolate, together with microcrystalline cellulose, lactose and aerosil and finally with magnesium stearate. The obtained mixture is compressed and the tablets are coated with a coating dispersion formed by traditional coating agents.

### Example 2.

Quantitative composition:

| | % by weight |
|---|---|
| Quetiapine hemifumarate | 37 |
| Lactose monohydrate | 20 |
| Microcrystalline cellulose (Avicel PH102) | 20 |
| Povidone (K-25) | 3 |
| Na starch glycolate type A (Primojel) | 11 |
| Glyceryl behenate | 5 |
| Anhydrous colloidal silica (Aerosil) | 0.3 |
| Magnesium stearate | 1 |
| Purified water* | 31* |
| | |
| Coating dispersion | 3** |

| | |
|---|---|
| * solvent which disappears during the manufacturing process. ** dry residue | |

Detailed Description of the manufacturing process:
Quetiapine hemifumarate is mixed with povidone, 50% of the total sodium starch glycolate and 50% of the total microcrystalline cellulose. The mixture is granulated in a low shear mixer with purified water, dried and sieved. The obtained granules are coated with glyceryl behenate by mixing. The coated granules are mixed with the remaining 50% of microcrystalline cellulose and sodium starch glycolate, together with lactose and aerosil and finally with magnesium stearate. The obtained mixture is compressed and the tablets are coated with a coating dispersion formed by traditional coating agents.

### Example 3.

Quantitative composition:

| | % by weight |
|---|---|
| Quetiapine hemifumarate | 37 |
| Lactose monohydrate | 22 |
| Microcrystalline cellulose (Avicel PH102) | 22 |
| Povidone (K-25) | 3 |
| Na starch glycolate type A (Primojel) | 7 |
| Glyceryl behenate | 5 |
| Anhydrous colloidal silica (Aerosil) | 0.3 |
| Magnesium stearate | 1 |
| Purified water* | 29* |
| Coating dispersion | 3** |

| | |
|---|---|
| * solvent which disappears during the manufacturing process. ** dry residue | |

Detailed Description of the manufacturing process:
Quetiapine hemifumarate is mixed with povidone, 50% of the total sodium starch glycolate and 50% of the total microcrystalline cellulose. The mixture is granulated in a low shear mixer with purified water, dried and sieved. The obtained granules are coated with glyceryl behenate by mixing. The coated granules are mixed with the remaining 50% of microcrystalline cellulose and sodium starch glycolate, together with lactose and aerosil and finally with magnesium stearate. The obtained mixture is compressed and the tablets are coated with a coating dispersion formed by traditional coating agents.

### Example 4.

| Quantitative composition: | |
|---|---|
| | % by weight |
| Quetiapine hemifumarate | 58 |
| Lactose monohydrate | 16 |
| Microcrystalline cellulose (Avicel PH102) | 16 |
| Povidone (K-25) | 5 |
| Na starch glycolate type A (Primojel) | 5 |
| Magnesium stearate | 1 |
| Purified water* | 38* (Meth. A) |
| | 43* (Meth. B) |

| | |
|---|---|
| * solvent which disappears during the manufacturing process. | |

The formula described in example 4 was manufactured by two different methods, one wherein the granulation is carried out using a high shear mixer (Method A) and other wherein a low shear mixer is used (Method B).

The dissolution profiles of the obtained tablets were determined in hydrochloric acid (900 ml, Ph. Eur. paddle apparatus, 50 rpm). The obtained results are indicated in the following table:

| | % DISSOLVED | |
|---|---|---|
| TIME | Method A | Method B |
| 15 | 37 | 97 |
| 30 | 72 | 101 |
| 45 | 90 | 101 |
| 60 | 99 | 103 |

By using a low shear mixer during the granulation process, a faster dissolution profile is obtained, allowing a total dissolution of the tablet at least 30 minutes before than the tablet containing granules manufactured with a high shear mixer.

### Example 5.

Quantitative composition:

| | Formula A (%) | Formula B (%) |
|---|---|---|
| Quetiapine hemifumarate | 38 | 38 |
| Lactose monohydrate | 20 | 18 |
| Microcrystalline cellulose (Avicel PH102) | 21 | 19 |
| Dibasic calcium phosphate | 1 | 1 |
| Povidone (K-25) | 3 | 3 |
| Na starch glycolate type A (Primojel) | 15 | 15 |
| Glyceryl behenate | -- | 5 |
| Magnesium stearate | 1 | 1 |
| Purified water* | 28* | 28* |

| | | |
|---|---|---|
| * solvent which disappears during the manufacturing process. | | |

Formulas A and B were manufactured following the method described in Example 1. The difference between both formulas is in the coating of the obtained granules; in formula B the granules are coated with glyceryl behenate by mixing, while in formula A the granules are not coated. Adherent compounds are obtained in the compression of formula A; the coating of the granules with glyceryl behenate solves the adhesion problems in the tablets.

## Claims

1. A granule formulation for the preparation of pharmaceutical compositions comprising:
a) a core comprising quetiapine or a pharmaceutically acceptable salt thereof as active ingredient and a binder agent, wherein the pharmaceutically acceptable salt is quetiapine fumarate; and
b) a coating layer comprising a lubricant agent, wherein the lubricant agent is glyceryl behenate.

2. The granule formulation according to claim 1 wherein the core further comprises a diluent agent and/or a disintegrant agent.

3. The granule formulation according to claims 1 or 2 wherein the binder agent is selected from the group comprising povidone, corn starch, hydroxypropylcellulose and copovidone, in particular wherein the binder agent is povidone K-25.

4. The granule formulation according to anyone of claims 2 to 3 wherein the diluent agent is selected from the group comprising microcrystalline cellulose, lactose monohydrate and dibasic calcium phosphate, in particular wherein the diluent agent is microcrystalline cellulose.

5. The granule formulation according to anyone of claims 2 to 4 wherein the disintegrant agent is selected from the group comprising sodium glicolate starch, crospovidone and sodium croscarmellose, in particular wherein the disintegrant agent is sodium starch glycolate type A (Primojel®).

6. The granule according to anyone of claims 1 to 5 wherein the active ingredient is quetiapine hemifumarate.

7. The granule according to anyone of claims 1 to 6 comprising:
a) a core comprising quetiapine hemifumarate, microcrystalline cellulose, sodium starch glycolate and povidone; and
b) a coating layer comprising glyceryl behenate.

8. The granule according to anyone of claims 1 to 7 wherein the quantity of lubricant agent is in a proportion between 5 and 25% by weight with respect to the total weight of the granule.

9. A process for the preparation of a granules as defined in anyone of claims 1 to 8 comprising:
a) providing quetiapine or a pharmaceutically acceptable salt thereof and, optionally mixing it with a disintegrant agent and/or a diluent agent, wherein the pharmaceutically acceptable salt is quetiapine fumarate;
b) adding to the quetiapine fumarate or to the mixture obtained in step a) a binder agent;
c) adding a solvent to the mixture obtained in step b), or optionally, adding a solution or suspension containing a binder and a solvent to the quetiapine fumarate or to the mixture obtained in step a), thus suppressing step b);
d) wet granulating the mixture obtained in step c);
e) drying the granules obtained in step d);
f) sieving the dried granules obtained in step e); and
g) coating the granules with a lubricant agent, wherein the lubricant agent is glyceryl behenate.

10. The process according to claim 9 wherein the solvent used in step c) is water, a hydroalcoholic mixture or one or more alcohols.

11. The process according to claims 9 or 10 wherein the wet granulating is carried out in a low shear mixer.

12. The process according to anyone of claims 9 to 11 wherein the drying step e) provides granules with a humidity content lower than 5%, preferably lower than 3%.

13. Use of a granules as defined in anyone of claims 1 to 8 for the elaboration of pharmaceutical compositions.

14. A pharmaceutical composition comprising granules as defined in any of claims 1 to 8, optionally in combination with one or more pharmaceutically acceptable excipients.

15. The composition according to claim 14 which is an immediate release composition.

16. The composition according to claim 14 which is a sustained release composition.

17. The pharmaceutical composition according to anyone of claims 14 to 16 in the form of a tablet.

18. An immediate release tablet which comprises granules as defined in anyone of claims 1 to 8 wherein the quantity of the lubricant agent is between 5 and 10% by weight with respect to the total weight of the granule.

19. A sustained release tablet which comprises a granule formulation as defined in anyone of claims 1 to 8 wherein the quantity of the lubricant agent is between 15 and 25% by weight with respect to the total weight of the granule formulation.

20. A process for the preparation of a tablet as defined in any of claims 18 or 19 comprising:
a) preparing granules as defined in claims 9 to 12;
b) optionally mixing the granules obtained in step a) with one or more pharmaceutically acceptable excipients;
c) tableting the mixture obtained in step b); and
d) coating the tablets obtained in step c).

## Patentansprüche

1. Granulatzubereitung für die Herstellung pharmazeutischer Zusammensetzungen, welche umfasst:
a) einen Kern umfassend Quetiapin oder ein pharmazeutisch annehmbares Salz desselben als aktiven Bestandteil und ein Bindemittel, wobei das pharmazeutisch annehmbare Salz Quetiapinfumarat ist; und
b) eine Beschichtungsschicht umfassend ein Gleitmittel, wobei das Gleitmittel Glycerylbehenat ist.

2. Granulatzubereitung nach Anspruch 1, wobei der Kern ferner ein Streckmittel und/oder ein Zerfallsmittel umfasst.

3. Granulatzubereitung nach Anspruch 1 oder 2, wobei das Bindemittel ausgewählt ist aus der Gruppe umfassend Povidon, Maisstärke, Hydroxypropylcellulose und Copovidon, insbesondere ist das Bindemittel Povidon K-25.

4. Granulatzubereitung nach einem der Ansprüche 2 bis 3, wobei das Streckmittel ausgewählt ist aus der Gruppe umfassend mikrokristalline Cellulose, Laktosemonohydrat und zweibasiges Calciumphosphat, insbesondere ist das Streckmittel mikrokristalline Cellulose.

5. Granulatzubereitung nach einem der Ansprüche 2 bis 4, wobei das Zerfallsmittel ausgewählt ist aus der Gruppe umfassend Natriumglycolatstärke, Crospovidon u n d Natriumcroscarmellose, insbesondere ist das Zerfallsmittel Natriumstärkeglycolat des Typs A (Primojel®).

6. Granulat nach einem der Ansprüche 1 bis 5, wobei der aktive Bestandteil Quetiapinhemifumarat ist.

7. Granulat nach einem der Ansprüche 1 bis 6, umfassend:
a) einen Kern umfassend Quetiapinhemifumarat, mikrokristalline Cellulose, Natriumstärkeglycolat und Povidon; und
b) eine Beschichtungsschicht umfassend Glycerylbehenat.

8. Granulat nach einem der Ansprüche 1 bis 7, wobei die Menge an Gleitmittel in einem Anteil zwischen 5 und 25 Gewichtsprozent in Bezug auf das Gesamtgewicht des Granulats vorliegt.

9. Verfahren zur Herstellung eines Granulats, wie es in einem der Ansprüche 1 bis 8 definiert ist, welches umfasst:
a) Bereitstellen von Quetiapin oder eines pharmazeutisch annehmbaren Salzes desselben und optional Mischen desselben mit einem Zerfallsmittel und/oder einem Streckmittel, wobei das pharmazeutisch annehmbare Salz Quetiapinfumarat ist;
b) Zufügen eines Bindemittels zum Quetiapinfumarat oder zu der in Schritt a) erhaltenen Mischung;
c) Zufügen eines Lösungsmittels zu der in Schritt b) erhaltenen Mischung, oder optional Zufügen einer Lösung oder Suspension enthaltend ein Bindemittel und ein Lösungsmittel zum Quetiapinfumarat oder zu der in Schritt a) erhaltenen Mischung, somit ausblendend Schritt b);
d) Nassgranulieren der in Schritt c) erhaltenen Mischung;
e) Trocknen des in Schritt d) erhaltenen Granulats;
f) Sieben des in Schritt e) erhaltenen getrockneten Granulats; und
g) Beschichten des Granulats mit einem Gleitmittel, wobei das Gleitmittel Glycerylbehenat ist.

10. Verfahren nach Anspruch 9, wobei das in Schritt c) verwendete Lösungsmittel Wasser, eine hydroalkoholische Mischung oder ein oder mehrere Alkohole ist.

11. Verfahren nach Anspruch 9 oder 10, wobei das Nassgranulieren in einem Niederschermischer durchgeführt wird.

12. Verfahren nach einem der Ansprüche 9 bis 11, wobei der Trocknungsschritt e) Granulate mit einem Feuchtigkeitsgehalt von kleiner als 5%, bevorzugt kleiner als 3%, bereitstellt.

13. Verwendung eines Granulats, wie es in einem der Ansprüche 1 bis 8 definiert ist, für die Zubereitung pharmazeutischer Zusammensetzungen.

14. Pharmazeutische Zusammensetzung umfassend Granulate, wie sie in einem der Ansprüche 1 bis 8 definiert sind, optional in Kombination mit einem oder mehreren pharmazeutisch annehmbaren Hilfsstoffe.

15. Zusammensetzung nach Anspruch 14, welches eine Zusammensetzung zur sofortigen Freisetzung ist.

16. Zusammensetzung nach Anspruch 14, welches eine Zusammensetzung zur verzögerten Freisetzung ist.

17. Pharmazeutische Zusammensetzung nach einem der Ansprüche 14 bis 16 in der Form einer Tablette.

18. Tablette zur sofortigen Freisetzung, welche Granulate, wie sie in einem der Ansprüche 1 bis 8 definiert sind, umfasst, wobei die Menge des Gleitmittels zwischen 5 und 10 Gewichtsprozent in Bezug auf das Gesamtgewicht des Granulats ist.

19. Tablette zur verzögerten Freisetzung, welche eine Granulatzubereitung, wie sie in einem der Ansprüche 1 bis 8 definiert ist, umfasst, wobei die Menge des Gleitmittels zwischen 15 und 25 Gewichtsprozent in Bezug auf das Gesamtgewicht der Granulatzubereitung ist.

20. Verfahren für die Herstellung einer Tablette, wie sie in einem der Ansprüche 18 oder 19 definiert ist, umfassend:
a) Herstellen von Granulaten, wie es in Ansprüchen 9 bis 12 definiert ist;
b) optional Mischen der in Schritt a) erhaltenen Granulate mit einem oder mehreren pharmazeutisch annehmbaren Hilfsstoffe;
c) Tablettieren der in Schritt b) erhaltenen Mischung; und
d) Beschichten der in Schritt c) erhaltenen Tabletten.

## Revendications

1. Formulation de granulés pour la préparation de compositions pharmaceutiques comprenant:
a) un noyau comprenant de la quétiapine ou un sel pharmaceutiquement acceptable de celle-ci en tant que principe actif et un agent liant, le sel pharmaceutiquement acceptable étant le fumarate de quétiapine; et
b) une couche d'enrobage comprenant un agent lubrifiant, l'agent lubrifiant étant le béhénate de glycéryle.

2. Formulation de granulés selon la revendication 1, dans laquelle le noyau comprend en outre un agent diluant et/ou un agent désintégrant.

3. Formulation de granulés selon les revendications 1 ou 2, dans laquelle l'agent liant est sélectionné dans le groupe comprenant la povidone, l'amidon de maïs, l'hydroxypropylcellulose et la copovidone, en particulier dans laquelle l'agent liant est la povidone K-25.

4. Formulation de granulés selon l'une quelconque des revendications 2 à 3, dans laquelle l'agent diluant est sélectionné dans le groupe comprenant la cellulose microcristalline, le lactose monohydraté et le phosphate de calcium dibasique, en particulier dans laquelle l'agent diluant est la cellulose microcristalline.

5. Formulation de granulés selon l'une quelconque des revendications 2 à 4, dans laquelle l'agent désintégrant est sélectionné dans le groupe comprenant le glycolate d'amidon sodique, la crospovidone et la croscarmellose sodique, en particulier dans laquelle l'agent désintégrant est le glycolate d'amidon sodique de type A (Primojel®).

6. Granulé selon l'une quelconque des revendications 1 à 5, dans lequel le principe actif est l'hémifumarate de quétiapine.

7. Granulé selon l'une quelconque des revendications 1 à 6 comprenant:
a) un noyau comprenant de l'hémifumarate de quétiapine, de la cellulose microcristalline, du glycolate d'amidon sodique et de la povidone; et
b) une couche d'enrobage comprenant du béhénate de glycéryle.

8. Granulé selon l'une quelconque des revendications 1 à 7, dans lequel la quantité d'agent lubrifiant se situe dans une proportion entre 5 et 25 % en poids par rapport au poids total du granulé.

9. Procédé de préparation de granulés tels que définis dans l'une quelconque des revendications 1 à 8 comprenant les étapes consistant à:
a) fournir de la quétiapine ou un sel pharmaceutiquement acceptable de celle-ci et, éventuellement la mélanger avec un agent désintégrant et/ou un agent diluant, le sel pharmaceutiquement acceptable étant le fumarate de quétiapine;
b) ajouter au fumarate de quétiapine ou au mélange obtenu à l'étape a) un agent liant;
c) ajouter un solvant au mélange obtenu à l'étape b), ou éventuellement, ajouter une solution ou une suspension contenant un liant et un solvant au fumarate de quétiapine ou au mélange obtenu à l'étape a), supprimant ainsi l'étape b);
d) granuler par voie humide le mélange obtenu à l'étape c);
e) sécher les granulés obtenus à l'étape d);
f) tamiser les granulés séchés obtenus à l'étape e); et
g) enrober les granulés avec un agent lubrifiant, l'agent lubrifiant étant le béhénate de glycéryle.

10. Procédé selon la revendication 9, dans lequel le solvant utilisé à l'étape c) est l'eau, un mélange hydroalcoolique ou un ou plusieurs alcools.

11. Procédé selon les revendications 9 ou 10, dans lequel la granulation par voie humide est réalisée dans un mélangeur à faible cisaillement.

12. Procédé selon l'une quelconque des revendications 9 à 11, dans lequel l'étape de séchage e) fournit des granulés avec une teneur en humidité inférieure à 5 %, de préférence inférieure à 3 %.

13. Utilisation de granulés tels que définis dans l'une quelconque des revendications 1 à 8 pour l'élaboration de compositions pharmaceutiques.

14. Composition pharmaceutique comprenant des granulés tels que définis dans l'une quelconque des revendications 1 à 8, facultativement en combinaison avec un ou plusieurs excipients pharmaceutiquement acceptables.

15. Composition selon la revendication 14 qui est une composition à libération immédiate.

16. Composition selon la revendication 14 qui est une composition à libération prolongée.

17. Composition pharmaceutique selon l'une quelconque des revendications 14 à 16 sous la forme d'un comprimé.

18. Comprimé à libération immédiate qui comprend des granulés tels que définis dans l'une quelconque des revendications 1 à 8, dans lequel la quantité de l'agent lubrifiant se situe entre 5 et 10 % en poids par rapport au poids total du granulé.

19. Comprimé à libération prolongée qui comprend une formulation de granulés telle que définie dans l'une quelconque des revendications 1 à 8, dans lequel la quantité de l'agent lubrifiant se situe entre 15 et 25 % en poids par rapport au poids total de la formulation de granulés.

20. Procédé de préparation d'un comprimé tel que défini dans l'une quelconque des revendications 18 ou 19 comprenant les étapes consistant à:
a) préparer des granulés tel que défini dans les revendications 9 à 12;
b) mélanger facultativement les granulés obtenus à l'étape a) avec un ou plusieurs excipients pharmaceutiquement acceptables;
c) fabriquer des comprimés avec le mélange obtenu à l'étape b) et;
d) enrober les comprimés obtenus à l'étape c).
